# EUROPEAN PATENT APPLICATION

(11) **EP 4 338 796 A1**
(43) Date of publication of application: **20.03.2024**
(21) Application number: 22806889.6
(22) Date of filing: 05.05.2022
(51) Int. Cl.: A61P 11/00, H05H 1/00, A61K 41/00, A61K 35/00

(54) **PLASMA-ACTIVATED MEDIA FOR USE THEREOF IN THE TREATMENT OF RESPIRATORY DISEASES**

(30) Priority: 13.05.2021 ES 202130439
(71) Applicant: MEDICAL PLASMAS, S.L., 31110 Noain Navarra (ES)
(72) Inventor: GÓMEZ CASADO, Eduardo, 28040 Madrid (ES); BRUN, Alejandro, 28040 Madrid (ES); MORENO FERNÁNDEZ, Sandra, 28040 Madrid (ES); MEGÍA MACÍAS, Ana María, 13500 Puertollano (CIUDAD REAL) (ES); CORTÁZAR PÉREZ, Osvaldo Daniel, 13500 Puertollano (CIUDAD REAL) (ES)
(74) Representative: Temiño Ceniceros, Ignacio
(86) International application number: PCT/ES2022/070275
(87) International publication number: WO 2022/238603

(57) **Abstract**

The proposed invention relates to plasma-activated liquid media for use thereof in the treatment of diseases, whether viral and/or bacterial (including antibiotic-resistant bacteria), of the upper and lower respiratory tracts in humans and animals. These media exhibit an antiviral and antibacterial activity based on the oxidative stress that they cause in the microorganisms and cells affected by the same. Said oxidative stress is caused by reactive oxygen and nitrogen molecule species dissolved in the plasma-activated medium and is tolerated by the healthy cells, but it inactivates said harmful microorganisms. The proposed use is useful for fighting viral and/or bacterial respiratory infections.

## Description

### TECHNICAL SECTOR

A61P 11/00. Medical or veterinary science, hygiene. Specific therapeutic activity of chemical compounds or medicinal preparations. Drugs for the treatment of disorders of the respiratory system.

### BACKGROUND OF THE INVENTION

In physics, plasma is defined as the fourth state of matter. It is obtained by providing enough energy to a gas so that a significant part of its molecules or atoms become ionised. There are many examples of plasmas in nature, ranging from the northern lights and lightning in storms to stars with a ring in said state, such as our sun. Furthermore, there are examples of technology such as plasmas in fluorescent lamps and plasma screens, among others. In general, the temperature of plasmas is too high, thus making them incompatible with living tissue. However, there are also "cold atmospheric" plasmas (hereinafter CAP), which have a low enough temperature that allows them to interact with living cells without damaging them. These plasmas have disinfectant and regenerative properties that make them of great interest in medical, veterinary and food technology applications, where they are able to inactivate bacteria, fungi and viruses without damaging healthy tissue (LAROUSSI, 2018; WELTMANN *et al.,* 2019). The basic principle of the CAP therapeutic action is summarised in the concept of "oxidative stress" caused by reactive oxygen and nitrogen species (hereinafter RONS) that are present in plasma. The oxidative stress caused by CAP is well tolerated by healthy cells, but it destroys bacteria, fungi and viruses (VON WOEDTKE *et al.,* 2019).

Moreover, "Plasma-Activated Media" (hereinafter PAM) have been developed. These media are primarily liquids that have been in direct contact with a CAP. One part of the RONS in the plasma are absorbed by these media, remaining in dilution for sufficiently long periods of time so that they can be useful in biological and medical applications. One example is distilled water or any of its saline solutions for medical use that have been in direct contact with a plasma.

PAM have antiviral, antibacterial, antifungal and antitumour properties that have aroused the interest of the scientific community as can be seen in the extensive literature available, where the most used media are saline solution or physiological saline and distilled water, also known as PAW (Plasma-Activated Water) (ZHANG *et al.,* 2013; SHAW *et al.,* 2018).

The disinfectant properties of PAM have drawn the attention of medical, agriculture and food technology research communities. The active component of its action is due to its ability to induce oxidative stress in cells. This concept can be summed up by saying that PAM are an indirect way of applying the properties of a cold atmospheric plasma or CAP, but while using a liquid as an intermediate. This makes it possible to reach areas of the human body and animal body that could not be reached with a cold atmospheric plasma generator.

Patenting PAM for use thereof, in liquid and/or aerosol form, in the treatment of viral and/or bacterial diseases (including those caused by antibiotic-resistant bacteria) of the respiratory system in humans and animals is proposed.

Both cold atmospheric plasmas, along with a wide variety of devices for generating them, and PAM and their associated devices for producing them, have been widely disseminated, forming part of the current state of the art. None of these objects and associated devices are claimed in this patent.

However, PAM used in liquid form as a mouthwash, gargle solution and/or aerosol in the treatment of diseases of the respiratory system in humans and animals are novel and patentable, this being the central topic of this patent.

The closest background of the invention is the document by Zhitong Chen and Richard E. Wirtz entitled: "Cold atmospheric plasma for COVID-19", which is worth noting since it proposes the use of CAP and PAM for disinfecting surfaces in hospitals and health centres in the context of the COVID-19 pandemic, replacing the usual alcohol solutions (CHEN and WIRZ, 2020, page 4 and 5). The same document highlights the anti-inflammatory action of reactive nitrogen molecule species (RNS) present in a CAP in a gaseous form, mainly nitrous oxide NO, and its potential for treating patients with COVID-19. However, the use of PAM as an inhaled aerosol for treating patients with COVID-19 or other respiratory diseases is not specified anywhere. Furthermore, this document does not disclose assay data, that is either its own or from a third party, on the effectiveness of PAM in inactivating SARS-CoV-2 or other viruses.

It is important to highlight, in favour of the inventive step of this patent, that in addition to the originality of the proposal, the very experimental results of the effectiveness of PAM as an antiviral for the case of the PR8 H1N1 virus responsible for influenza or the common flu, SARS-CoV-2 of COVID-19, and as an antibacterial for E. *coli* and *Pseudomonas aeruginosa* are presented. In the preferred embodiment of the invention section, the laboratory work carried out by the authors, as well as the characterisation of the PAM used are disclosed.

### DESCRIPTION OF THE INVENTION

The present invention relates to PAM for use thereof in the treatment of respiratory diseases in humans and animals.

The plasma-activated medium is prepared by means of a device specially designed for this purpose, which is not the object of this patent. This device transfers a certain concentration of reactive oxygen and nitrogen species or RONS, which are present in a cold atmospheric air plasma or CAP, to the liquid medium to be activated, for a suitable time of direct exposure. This concentration of dissolved RONS remains in the medium and is responsible for the therapeutic action thereof.

The plasma-activated medium can be used in liquid form for mouthwashes and gargle solutions and also as a spray or aerosol in the respiratory system of the patient suffering from a respiratory disease. In the case of use as an aerosol, it can be used naturally (by inhalation) or in a forced manner by means of an artificial mechanical system. In the first case, the patient inhales the aerosol of the plasma-activated medium on their own using a mask. In the second case, patients are intubated and assisted with mechanical ventilation, wherein the aerosol of the plasma-activated medium is introduced as part of the air or oxygen flow they breathe.

The present invention has the following features:
a. It is applied to patients who suffer from respiratory diseases and/or viral and/or bacterial respiratory infections.
b. It is applied directly as a mouthwash, gargle solution or aerosol/spray.
c. The active component on which the proposed use is based is the oxidative stress caused by reactive oxygen and nitrogen radical molecules, which are dissolved in the plasma-activated medium on the cells and microorganisms that are part of or are active in the tissue reached by the medium. This oxidative stress is well tolerated by healthy cells, but it inactivates viruses and/or bacteria.
d. It does not produce resistance in bacteria.

### DESCRIPTION OF THE FIGURES

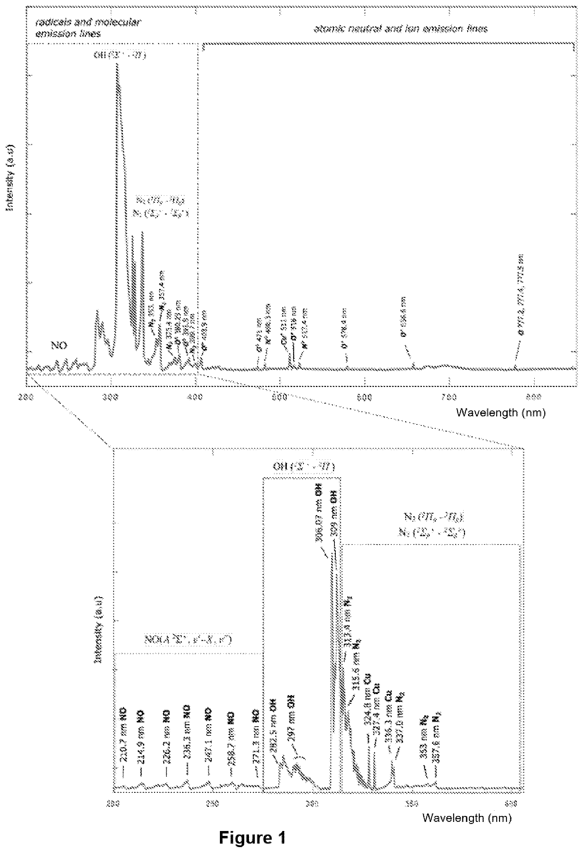

**Figure 1****:** UV-VIS spectrum (200-800 nm) and UV spectrum (200-400 nm) obtained from the light emitted by the plasma during the activation of PAW to monitor the presence of RONS during the process; these spectra are part of quality control during PAW production.

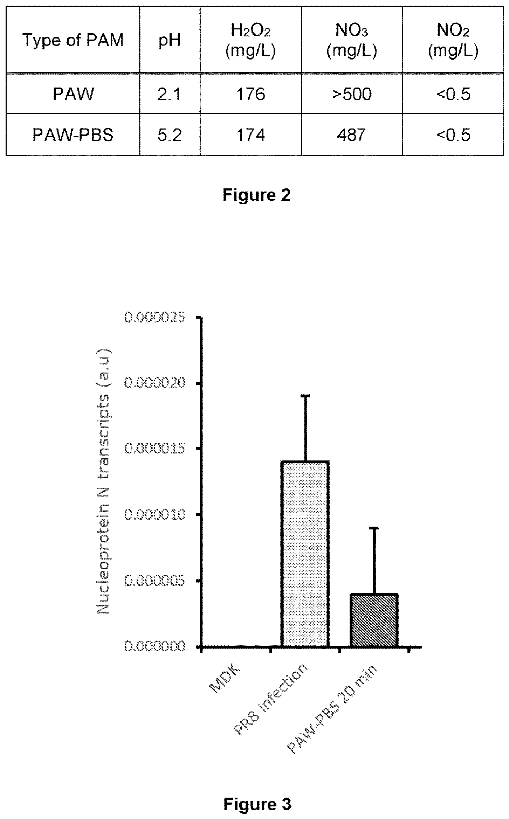

**Figure 2****:** Table of final pH values and RONS concentrations in mg/L dissolved in the PAW, obtained by the action of the plasma, which were used in the assays. Anions: hydrogen peroxide (H₂O₂⁻), nitrate (NO₃⁻) and nitrite (NO₂⁻).
**Figure 3****:** Results of inhibition on PR8 H1N1 virus replication in the canine kidney MDCK cell line obtained after infection with PR8 and subsequent treatment with PAW buffered with PBS (PAW-PBS). The results show the level of nucleoprotein (N) messenger RNA transcripts of the PR8 virus measured by quantitative PCR (qPCR). The first column represents the reference sample, the second column represents the sample of cells infected with PR8 (PR8 Infection) and the third column represents the sample of cells infected with PR8 that were treated with PAW-PBS for 20 minutes of exposure (PAW-PBS 20min).

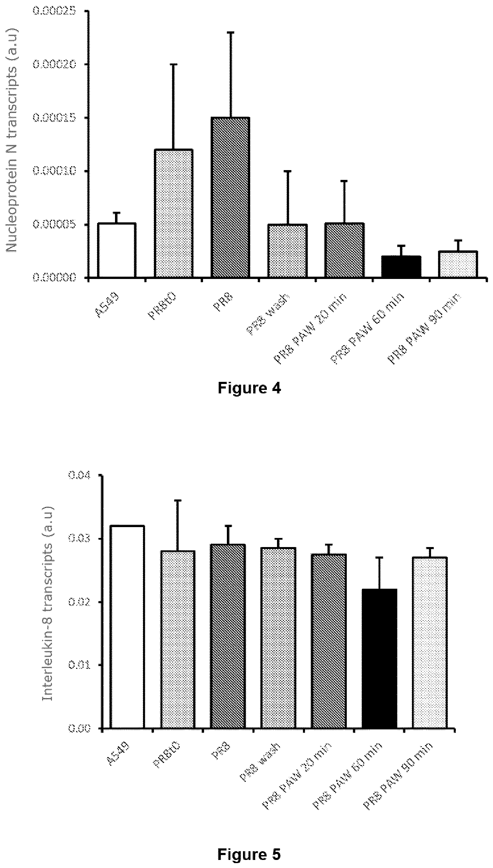

**Figure 4****:** Results of inhibition on the internalisation of the PR8 virus in an A549 human lung cell line subjected to infection for one hour and subsequently treated with PAW-PBS for 20, 60 and 90 minutes. The amount of nucleoprotein (N) messenger RNA transcripts of the virus was measured by quantitative PCR (qPCR). The first three columns represent the reference cells (A549), the initial adsorption level of the virus (PR8t0) and the infection level after one hour of adsorption (PR8), respectively. The following columns show the reduced adsorption levels obtained with the PAW treatments used. The PR8 2 wash column shows the result of two consecutive washes, the PR8 PAW 20 min column shows washing lasting 20 minutes and the following columns show washing lasting 60 and 90 minutes, respectively.
**Figure 5****:** Study of inflammation caused by PAW treatment in an A549 human lung cell line. The amount of interleukin-8 messenger RNA transcripts, a precursor cytokine of inflammation that makes it possible to establish whether the use of PAM triggers inflammatory processes, was measured by qPCR (BAGGIOLINI AND CLARK-LEWIS, 1992). The values obtained with the same order of data mentioned in Figure 4 are shown.

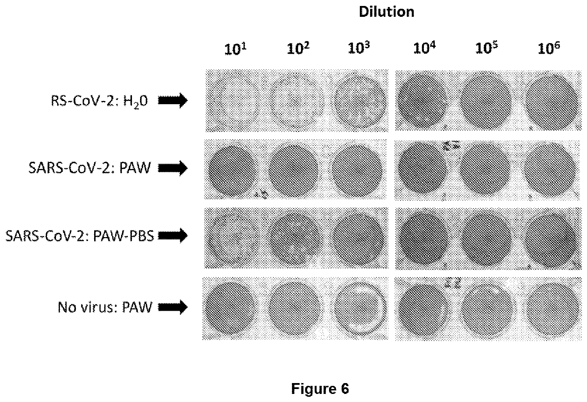

**Figure 6****:** Results of a series of assays with different SARS-CoV-2 dilutions in distilled water and deposited on a monolayer of the VERO E6 monkey kidney cell line. A top view of 24 wells is shown, where a monolayer of cells that were subjected to dilutions of the virus from 10¹ to 10⁶ was deposited. The first (top) row shows the effect of each dilution on the cells. The light colour represents cell death due to viral activity and the dark colour represents healthy cells. The devastating effect of the virus on cells is clearly seen in the first two concentrations corresponding to 1/10 and 1/100, respectively. The second row shows the effect of the SARS-CoV-2 dilutions on the cell bed when previously incubated with PAW for 60 minutes. Total disappearance of virus infectivity is observed. The third row shows the effect of a SARS-CoV-2 dilution on PAW-PBS under the same incubation conditions described.

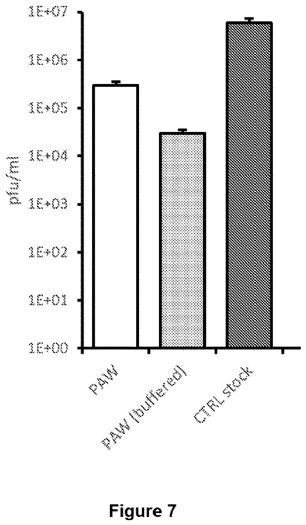

**Figure 7****:** Results of the action of washing with PAW and PAW-PBS on the amount of SARS-CoV-2 virus adsorbed on the VERO E6 cells for one hour. Noticeable effectiveness in reducing the amount of virus absorbed by the cells compared to the control column (CTRL stock) is observed.

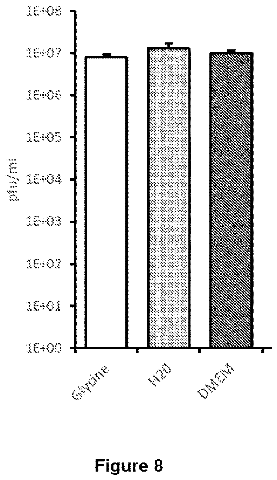

**Figure 8****:** Results of the study on the influence of the pH of PAW on its antiviral activity. The measurements of SARS-CoV-2 viral activity with distilled water (pH=5), 0.1 M Glycine (pH=2.7) and DMEM (pH=7) are shown. It is observed that the effect of the pH of the solution on virus infectivity is not significant.

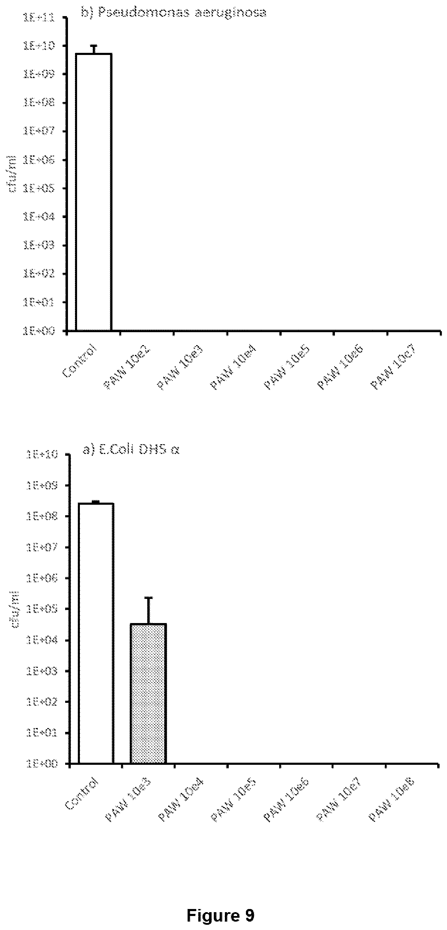

**Figure 9**: (a) Results of exposure of *Escherichia coli* DH5α. Serial dilutions of 10¹ to 10⁸ were performed in pure PAW and incubated for 2 hours to observe bacterial survival. The *E*. *coli* control indicates an average of 2.6×10⁸ cfu/mL, while the dilutions treated with PAW were negative. **(b)** Results of exposure of *Pseudomonas aeruginosa.* Serial dilutions of 10¹ to 10⁹ were performed in pure PAW and incubated for 2 hours to observe bacterial survival. The *P. aeruginosa* control indicates 5.15×10⁹ cfu/mL, while the dilutions treated with PAW were only positive at the 1/10 dilution and negative from the 1/100 dilution.

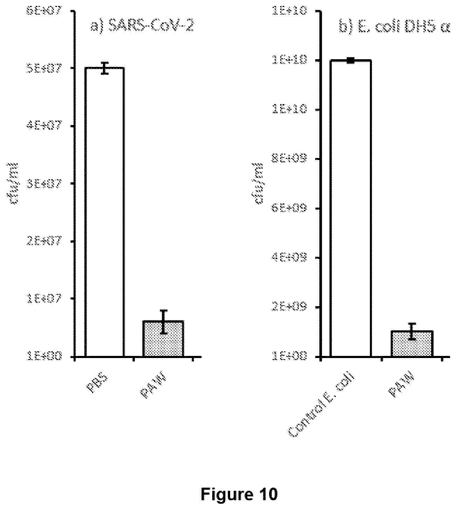

**Figure 10****:** Results from assays exposing virus and bacteria samples to a PAW spray or aerosol inside a chamber specially designed for this purpose. Figure 10a) shows the data obtained with nebulising VERO E6 cell line samples infected with SARS-CoV-2. Figure 10b) shows the data from nebulising samples of the *E*. *coli* DH5α bacteria. Both cases show a drop of one order of magnitude in the activity.

### PREFERRED EMBODIMENT OF THE INVENTION

The use of deionised water activated with PAW plasma as an antiviral against two viruses with a high social impact: PR8 H1N1 influenza or common flu virus and SARS-CoV-2 responsible for COVID-19, is described below as a preferred embodiment of the invention and with examples. Furthermore, the results of antibacterial activity assays with *E*. *coli* DH5α and *Pseudomonas aeruginosa* are shown, the latter being very common in respiratory tract infections.

The viruses used spread mainly dissolved in the microdroplets that form the aerosol expelled by any person through their mouth and nose when breathing, talking, singing or screaming. Once the contaminated microdroplets are in suspension, they can be inhaled by healthy people, coming into contact with the mucous membranes of their respiratory tract and infecting them. In the specific case of SARS-CoV-2, the rate of multiplication in the upper respiratory tract during the first week of symptoms is one thousand times higher than its predecessor SARS-CoV-1 (WOLFEL *et al.,* 2020).

The results presented are from *in-vitro* assays carried out at facilities of the National Centre for Agricultural and Food Research and Technology (INIA) located in Madrid and under the Higher Council of Scientific Research of Spain (CSIC). Assays with SARS-CoV-2 were carried out at the High Security Biological Laboratory of the Animal Health Research Centre (CISA), while assays with PR8 and bacteria were carried out at its Biotechnology Department. PAW was produced at the facilities of the company ION BIOTEC S.L. in Puertollano, Ciudad Real. Both INIA-CSIC as an institution and ION BIOTEC S.L. as a company are legal entities in Spain and co-proprietors of the proposed patent.

The states that these viruses can take during their life cycle are taken into consideration:
(a) Dissolved in respiratory aerosol microdroplets.
(b) Penetrating healthy cells of the respiratory mucous membrane of an infected person.
(c) Inside the infected cells and using them to multiply.
(d) Leaving infected cells to infect others and spread again.

### EXAMPLE 1: Plasma-Activated Water (PAW)

First, it is necessary to produce an amount of PAW with enough known and reproducible RONS concentrations. Figure 1 shows two spectra of the light emitted by the air plasma while it interacts with the surface of the deionised water during the production of PAW used in all assays described in this section. The spectrum at the top of Figure 1 is a record in the UV and visible wavelength range between 200 and 800 nm, while the spectrum at the bottom was obtained with a UV spectrometer having a higher resolution between 200 and 400 nm. Both show the presence of the characteristic spectral lines emitted by the reactive oxygen and nitrogen species that provide the properties required for the use at hand. These spectral measurements are part of quality control in PAW production and were carried out on all batches used in the assays referred to herein. In addition to these records, the concentrations of the most important ions in PAW were determined once it was produced. The concentrations of the anions of nitrate (NO3⁻), nitrite (NO2⁻) and hydrogen peroxide (H₂O₂⁻) in mg/L were measured, these concentrations being the most stable and providing the desired therapeutic characteristics. These anions are the most relevant part of the ion population that was previously called RONS. Furthermore, the pH of the samples obtained was determined. These records were made using a system of colorimetric strips that are immersed for one second in the PAW and their colour change is immediately measured with specific spectrophotometry equipment. This makes it possible to obtain objective values of the concentrations and the pH.

Figure 2 shows the ion concentrations of the PAW that were used in the assays: PAW as previously defined and PAW-PBS, a dilution of PAW buffered with PBS. The aim of the assays with PBS-buffered dilutions is to determine the influence of pH on the viruses studied.

It is important to highlight the high RONS concentration values available in the PAM produced and used herein compared to those published internationally. The publication by K. Kutasi and his colleagues (KUTASI *et al.,* 2019), page 2, Table 1, shows values that indicate the state of the art in the production of several typical PAM. In this case, with treated volumes of 200 ml, exposure times of 20 minutes and the concentrations of Figure 2, ion injection rates per unit of time are obtained that are more than one hundred times higher. This is an important contribution with regards to the viability of possible industrial production of PAW and its practical use.

### EXAMPLE 2: ASSAYS WITH PR8 H1N1 INFLUENZA OR COMMON FLU

### VIRUS

First, infection assays with PR8 virus, responsible for influenza or the common flu, were carried out by using a Madin-Darby canine kidney (MDCK) cell line. Once infected with the PR8 virus, they were left for 24 hours in a regime of free virus multiplication and subsequently washed with PAW-PBS for twenty minutes. Next, the amount of nucleoprotein (N) messenger RNA transcripts of the virus was measured by quantitative PCR (qPCR). Figure 3 shows the results of inhibition on replication. The three columns represent the reference sample (MDCK), the sample of cells infected with PR8 (PR8 Infection) and the sample of infected cells that were treated with PAW-PBS for 20 minutes of exposure (PAW-PBS 20m in). The antiviral activity of PAM used is observed because the level of nucleoprotein (N) transcripts decreases compared to the untreated sample.

Figure 4 shows the results of inhibition on the internalisation of the PR8 virus in a human lung cell line known as A549 subjected to infection for one hour and subsequently treated with PAW-PBS for 20, 60 and 90 minutes. Next, the amount of nucleoprotein messenger RNA transcripts of the virus was measured by quantitative PCR (qPCR). The first three columns represent the reference cells (A549), the initial adsorption level of the virus (PR8t0) and the adsorption level after one hour (PR8). The following columns show the reduced adsorption levels obtained with the treatments of PAM used. The PR8 2 wash column shows the result of two consecutive washes after adsorption, the PR8 PAW 20 min column shows washing lasting 20 minutes and the following columns show washing lasting 60 and 90 minutes, respectively. In all cases in which PAW-PBS is used, the inhibitory action of virus internalisation in the cells is observed.

### EXAMPLE 3: INFLAMMATION ASSAYS

In addition to these tests, a study of inflammation due to PAM treatment was carried out with the same human lung cell line. In this case, the amount of interleukin-8 messenger RNA transcripts, a precursor cytokine of inflammation that makes it possible to establish whether inflammatory processes occur (BAGGIOLINI AND CLARK-LEWIS, 1992), was measured by qPCR. This assay is important given its intended use for the purposes of this patent. Figure 5 shows the values obtained with the same order of data mentioned in Figure 4. It is shown that the inflammatory action of PAM on lung cells is not noticeable, meaning that it is deduced that PAW-PBS does not produce inflammation.

### EXAMPLE 4: ASSAYS WITH COVID-19 SARS-CoV-2 VIRUS

Moreover, and given the importance of the impact of SARS-CoV-2 in the context of the COVID-19 pandemic, assays were carried out with this virus.

Figure 6 shows the results of a series of assays with different SARS-CoV-2 dilutions in distilled water and deposited on a monolayer of the VERO E6 cell line (monkey kidney). Dilutions of the virus range from 10¹ to 10⁶. The first (top) row shows the effect of each dilution on the cells. The light colour represents cell death due to viral activity and the dark colour represents healthy cells. The devastating effect of the virus on cells is clearly seen in the first two concentrations corresponding to 1/10 and 1/100, respectively. The second row shows the effect of the SARS-CoV-2 dilutions in PAW on the cell bed for 60 minutes. Total disappearance of virus infectivity is observed. The third row shows the effect of a SARS-CoV-2 dilution in PAW-PBS. A much smaller effect of diminishing infectivity is observed. Lastly, the last row shows the effect of PAW on the bed of cells that have not been previously infected with the virus. Some marginal lifting effect on the cell layer is observed. This effect is not related to PAW and is due to the drying of the well which affects cell adhesion to those "bald" areas. In general, clear antiviral activity of PAW against SARS-CoV-2 is observed.

Furthermore, assays were carried out to determine the action of PAW on the amount of virus absorbed by the cells. VERO E6 cells were subjected to one hour of adsorption with SARS-CoV-2 and subsequently washed with PAW and PAW-PBS. Figure 7 shows the results obtained, showing noticeable effectiveness in reducing the amount of virus absorbed by the cells compared to the control column (CTRL stock).

### EXAMPLE 5: ASSAYS OF THE INFLUENCE OF ACIDITY, pH

In addition to these tests, assays were carried out to establish the influence of the pH of PAW on its antiviral activity. As previously mentioned, the active component of PAW is the concentration of RONS; therefore, it is important to determine whether the relatively low pH of PAW may be masking this effect. To that end, measurements of SARS-CoV-2 viral activity were carried out with distilled water (pH=5), 0.1 M Glycine (pH=2.7) and DMEM (pH=7). Figure 8 shows the results obtained, showing that the effect of the pH of the solution on virus infectivity does not justify the lower values observed in the previous assays.

### EXAMPLE 6: ASSAYS WITH BACTERIA, E. coli and Pseudomonas aeruginosa.

Considering the importance of bacterial infections in the respiratory tract, assays were carried out with two bacteria that are often the typical protagonists of these situations: *Escherichia coli* DH5α and *Pseudomonas aeruginosa.*

Starting from a stock of *Escherichia coli* DH5α, serial dilutions of 10¹ to 10⁸ were performed in pure PAW and incubated for 2 hours. Subsequently, they were plated on LB-Agar to observe bacterial survival. The *E*. *coli* control indicates an average of 2.6×10⁸ cfu/mL, while the dilutions treated with PAW were negative. Figure 9 a) shows these results.

Furthermore, an assay was carried out starting from a stock of *Pseudomonas aeruginosa,* with serial dilutions of 10¹ to 10⁹ in pure PAW and incubation for 2 hours. Subsequently, it was plated on LB-Agar to observe bacterial survival. The *P. aeruginosa* control indicates 5.15×10⁹ cfu/mL, while the dilutions treated with PAW were only positive at the 1/10 dilution and negative from the 1/100 dilution. This implies that PAW has destroyed 50 million bacteria. Figure 9 b) shows these results.

### EXAMPLE 7: ASSAYS WITH PLASMA-ACTIVATED WATER NEBULISATION

Assays exposing virus and bacteria samples to a PAW spray or aerosol inside a chamber specially designed for this purpose were performed. The chamber is made of transparent material and allows the aerosol to enter through a hose connected to an ultrasonic nebuliser. The wells with the samples to be treated can be accommodated in said chamber. The nebuliser is loaded with the liquid to be projected and is capable of producing a fine aerosol of droplets measuring up to 5 microns in air. This aerosol moves due to the pressure difference through the interior of the hose to the chamber where it is clearly observed that it penetrates said chamber where it is deposited on the surface of the samples. The chamber is equipped with filters that maintain the atmospheric pressure therein, but prevent mist from escaping to the outside.

This device was used to perform assays of exposure to nebulisation with PAW, wherein two cases are shown as examples:
a) Nebulisation of VERO E6 cell line samples infected with SARS-CoV-2.
b) Nebulisation of *E*. *coli* DH5α bacteria samples.

In the first case, a cell monolayer was used, as in previous assays, and in the second case, a volume of 100 microlitres of a solution with bacteria was used.

Figure 10 shows the results obtained in both cases. Both cases show a drop of one order of magnitude in the infective capacity of SARS-CoV-2 (Figure 10a) and in the colony forming capacity of *E*. *coli* DH5α (Figure 10b). This is a significant example of the antiviral and antibacterial activity of PAW applied as a spray or aerosol, the result of which allows the development of a treatment that uses this technique.

### LIST OF CITED REFERENCES

BAGGIOLINI, M. AND CLARK-LEWIS, I. (1992) `Interleukin-8, a chemotactic and inflammatory cytokine', FEBS Letters, 307(1), pp. 97-101. doi: 10.1016/0014-5793(92)80909-Z.
CHEN, Z. AND WIRZ, R. E. (2020) Cold atmospheric plasma for COVID-19. University of California, doi: 10.20944/preprints202004.0126.v1.
KUTASI, K. et al. (2019) 'Tuning the composition of plasma-activated water by a surface-wave microwave discharge and a kHz plasma jet', Plasma Sources Science and Technology, 28(9). doi: 10.1088/1361-6595/ab3c2f.
LAROUSSI, M. (2018) 'Plasma Medicine: A Brief Introduction', Plasma, 1(1), pp. 47-60. doi: 10.3390/plasma1010005.
SHAW, P. et al. (2018) 'Bacterial inactivation by plasma treated water enhanced by reactive nitrogen species', Scientific Reports, 8(1). doi: 10.1038/s41598-018-29549-6.
WELTMANN, K. D. et al. (2019) 'The future for plasma science and technology', Plasma Processes and Polymers, 16(1), pp. 1-29. doi: 10.1002/ppap.201800118.
VON WOEDTKE, T. et al. (2019) 'Plasma medicine: A field of applied redox biology', In Vivo, 33(4), pp. 1011-1026. doi: 10.21873/invivo.11570.
WOLFEL, R. et al. (2020) 'Virological assessment of hospitalized patients with COVID-2019', Nature, 3. doi: 10.1038/s41586-020-2196-x.
ZHANG, Q. et al. (2013) 'A study of oxidative stress induced by non-thermal plasma-activated water for bacterial damage', Applied Physics Letters, 102(20). doi: 10.1063/1.4807133.

## Claims

1. Plasma-activated liquid media, for use thereof in the treatment of diseases of the upper and lower respiratory tracts such as viral and/or bacterial infections (including those caused by antibiotic-resistant bacteria) in humans and animals.

2. The plasma-activated liquid media, for use thereof in the treatment of diseases of the upper and lower respiratory tracts such as viral and/or bacterial infections (including those caused by antibiotic-resistant bacteria) in humans and animals according to claim 1, **characterised by** the use thereof as a mouthwash and/or gargle solution.

3. The plasma-activated liquid media, for use thereof in the treatment of diseases of the upper and lower respiratory tracts such as viral and/or bacterial infections (including those caused by antibiotic-resistant bacteria) in humans and animals according to claim 1, **characterised by** the use thereof in the form of a respirable liquid that allows for liquid breathing in the lungs.

4. The plasma-activated liquid media, for use thereof in the treatment of diseases of the upper and lower respiratory tracts such as viral and/or bacterial infections (including those caused by antibiotic-resistant bacteria) in humans and animals according to claim 1, **characterised by** the use thereof as an aerosol or spray that is introduced into a patient's respiratory system either by natural inhalation or by artificial mechanical means.
